# EUROPEAN PATENT APPLICATION

(11) **EP 4 437 885 A1**
(43) Date of publication of application: **02.10.2024**
(21) Application number: 22910038.3
(22) Date of filing: 20.12.2022
(51) Int. Cl.: A24F 40/46, A24F 40/10

(54) **CERAMIC HEATING BODY, ATOMIZER AND AEROSOL GENERATING APPARATUS**

(30) Priority: 24.12.2021 CN 202111601495
(71) Applicant: Shenzhen First Union Technology Co., Ltd., Shenzhen, Guangdong 518000 (CN)
(72) Inventor: HUANG, Wenqiang, Shenzhen, Guangdong 51800 (CN); SU, Liangjie, Shenzhen, Guangdong 51800 (CN); LEI, Baoling, Shenzhen, Guangdong 51800 (CN); XU, Zhongli, Shenzhen, Guangdong 51800 (CN); LI, Yonghai, Shenzhen, Guangdong 51800 (CN)
(74) Representative: Jacob, Reuben Ellis
(86) International application number: PCT/CN2022/140452
(87) International publication number: WO 2023/116726

(57) **Abstract**

A ceramic heating body, an atomizer (100) and an aerosol generating apparatus, the atomizer (100) comprising: a housing; a porous liquid guide body (21), which is divided into two sections along the longitudinal direction thereof, the two sections being a first section (211) and a second section (212) respectively, the outer diameter of the first section (211) being smaller than the outer diameter of the second section (212), and the second section (212) forming a stepped surface (213) relative to the first section (211); a heating element (22), which is fixed onto the porous liquid guide body (21); a bracket (23), the porous liquid guide body (21) being accommodated in an accommodation cavity (235) of the bracket (23); and a first sealing element (242), at least a portion of the first sealing element (242) surrounding the outer side surface of the second section (212). The first sealing element (242) is arranged around the outer side surface of the second section (212) so as to provide a seal between the inner wall surface of the accommodating cavity (235) of the bracket (23) and the outer side surface of the second section (212), thereby preventing the leakage of a liquid matrix.

## Description

### CROSS-REFERENCE TO RELATED APPLICATIONS

This application claims priority to Chinese Patent Application No. 202111601495.X, entitled "CERAMIC HEATING BODY, ATOMIZER AND AEROSOL GENERATING APPARATUS" filed with the China National Intellectual Property Administration on December 24, 2021, which is incorporated herein by reference in its entirety.

### TECHNICAL FIELD

Embodiments of this application relate to the field of aerosol generating apparatus technologies, and in particular, to a ceramic heating body, an atomizer, and an aerosol generating apparatus.

### BACKGROUND

An aerosol generating apparatus includes an atomizer and a power supply assembly. A core assembly of the atomizer is an atomization assembly, and the atomization assembly mainly atomizes, through the atomization assembly, a liquid substrate stored inside the aerosol generating apparatus to generate an aerosol. There is a type of atomization assembly that is substantially in a shape of a tube, which mainly includes a ceramic heating body. The ceramic heating body includes a ceramic liquid guide element and a heating element fixed on the ceramic liquid guide element. In the conventional technology, the ceramic liquid guide element is usually configured as a hollow cylindrical structure with a consistent outer diameter. A part of an outer surface of the ceramic liquid guide element usually needs to be sealed and fixed in an inner cavity of a bracket by using liquid guide cotton. However, the liquid guide cotton often needs to be manually wound and fixed on an outer periphery of the ceramic liquid guide element, a requirement for a manual cotton winding operation is high, and although repeated training is performed, it is difficult for different operators to achieve consistency. As a result, in different ceramic heating bodies, a sealing degree of the liquid guide cotton on the ceramic guide liquid element is inconsistent, and a poor sealing effect may lead to leakage of the liquid substrate. In addition, due to a process of manual cotton winding, it is difficult to implement automate assembly of the atomization assembly.

### SUMMARY

To resolve a problem of a poor sealing effect of sealing a porous liquid guide element of an atomizer by using liquid guide cotton in the conventional technology.

Embodiments of this application provide an atomizer, including a housing, where a liquid storage cavity is defined and formed in the housing, and the liquid storage cavity is configured to store a liquid substrate; a porous liquid guide element, configured to include a hollow tubular structure, where the porous liquid guide element includes a first section and a second section that are arranged in a longitudinal direction of the porous liquid guide element, an outer diameter of the first section is less than an outer diameter of the second section, and a step surface is formed as a transition between the second section and the first section; a heating element, fixed on the ceramic liquid guide element, and configured to atomize the liquid substrate; a bracket, including an accommodating cavity, where the porous liquid guide element is accommodated in the accommodating cavity of the bracket, and a hole is provided on the bracket, and is configured to guide the liquid substrate to enter the accommodating cavity, to allow the liquid substrate to be absorbed by the porous liquid guide element; and a first sealing element, where at least a part of the first sealing element surrounds an outer side surface of the second section, to provide a seal between an inner wall surface of the accommodating cavity and the outer side surface of the second section.

In some embodiments, the heating element is arranged extending in the longitudinal direction of the porous liquid guide element, and the heating element is limited to extend in a longitudinal extension length range of the first section of the porous liquid guide element.

In some embodiments, a second sealing element is further included, where the second sealing element at least partially surrounds an outer side surface of an end portion of the first section facing away from the second section.

In some embodiments, a liquid guide cavity is defined and formed between an inner wall surface of the bracket and a part of an outer wall surface of the porous liquid guide element that is not surrounded by the first sealing element and the second sealing element, and the liquid substrate inside the liquid storage cavity enters the liquid guide cavity through the hole and then is transferred to the porous liquid guide element.

In some embodiments, a wall thickness of the first section is less than a wall thickness of the second section.

In some embodiments, the wall thickness of the first section ranges from 0.5 mm to 1.2 mm, and the wall thickness of the second section ranges from 1.2 mm to 3 mm.

In some embodiments, the accommodating cavity of the bracket includes a first part and a second part, an inner diameter of the first part is less than an inner diameter of the second part, the first part of the accommodating cavity is configured to accommodate the first section of the porous liquid guide element, and the second part of the accommodating cavity is configured to accommodate the second section of the porous liquid guide element.

In some embodiments, a first vent channel is formed between the first sealing element and the porous liquid guide element, and the first vent channel is configured to guide an external airflow to the liquid storage cavity.

In some embodiments, the first vent channel includes at least one groove extending on the outer side surface of the second section of the porous liquid guide element, and the groove is in communication with the liquid storage cavity.

In some embodiments, the groove extends non-linearly in the longitudinal direction.

In some embodiments, the groove extends in a substantially Z shape or S shape.

In some embodiments, the groove includes a first groove segment and a second groove segment in communication with each other, a width of the second groove segment is greater than a width of the first groove segment, and the second groove segment is in communication with the liquid storage cavity.

In some embodiments, the first vent channel further includes a first notch provided on the second sealing element, and the first notch is in communication with the groove and the liquid storage cavity.

In some embodiments, when the porous liquid guide element and the first sealing element are accommodated in the accommodating cavity, the first vent channel corresponds to a position of the hole on the bracket in the longitudinal direction.

In some embodiments, a first limiting structure is arranged on the first sealing element, and the first limiting structure is configured to provide a stop to prevent the porous liquid guide element from rotating relative to the first sealing element.

In some embodiments, a first fitting portion that matches the first limiting structure is further arranged on the porous liquid guide element, and the first fitting portion protrudes from the step surface.

In some embodiments, a third sealing element is further included that is at least partially accommodated in the bracket, and the third sealing element abuts against the porous liquid guide element in the longitudinal direction.

In some embodiments, the third sealing element further includes a second limiting portion, configured to cooperate with the bracket to prevent the third sealing element from rotating relative to the bracket.

In some embodiments, a second fitting portion that matches the second limiting portion is arranged on the bracket, and the second fitting portion includes a second notch.

In some embodiments, the third sealing element includes a cavity adjacent to an end surface of the second section, and the cavity is configured to receive condensate.

In some embodiments, the atomizer further includes an electrical connecting member that is electrically connected to the heating element, a baffle plate is arranged on the third sealing element, and the baffle plate extends from an end portion of the third sealing element toward the electrical connecting member.

In some embodiments, a vent hole is provided on the third sealing element.

In some embodiments, at least a part of a side surface of the third sealing element is concave, to enable an air guide cavity to be formed between the third sealing element and an inner wall of the bracket, and the air guide cavity is in communication with the vent hole.

In some embodiments, two ends of the heating element are connected to conductive pins, and the conductive pins extend out from inside of the porous liquid guide element, where an extension direction of each of the conductive pins is arranged non-parallel to an axis of the porous liquid guide element.

In some embodiments, the first sealing element includes a sleeve portion and a radial extension portion, and the radial extension portion covers the step surface.

Embodiments of this application further provide a ceramic heating body used in an aerosol generating apparatus, including a porous liquid guide element configured to store and transfer a liquid substrate and a heating element combined on the porous liquid guide element, where the porous liquid guide element is configured to include a hollow tubular structure, the porous liquid guide element includes a first section and a second section that are arranged in a longitudinal direction, an outer diameter of the first section is less than an outer diameter of the second section, and a step surface is formed as a transition between the second section and the first section; and the heating element is configured to atomize the liquid substrate. In some embodiments, at least one groove is provided on an outer wall of the porous liquid guide element.

Embodiments of this application further provide an aerosol generating apparatus, including the atomizer, and a power supply assembly that provides electric drive for the atomizer.

Beneficial effects of this application are: The porous liquid guide element includes the first section and the second section with different outer diameters, the step surface is formed between the second section and the first section, and the first sealing element is arranged surrounding the outer side surface of the second section, to provide a seal between the inner wall surface of the accommodating cavity of the bracket and the outer side surface of the second section, thereby preventing leakage of the liquid substrate. In comparison to the use of the liquid guide cotton for sealing, deformation of the first sealing element is small, and a more stable sealing effect is achieved. In addition, a process of manually winding the liquid guide cotton on the outer surface of the porous liquid guide element is avoided, so that assembly of the atomization assembly can be automated.

### BRIEF DESCRIPTION OF DRAWINGS

One or more embodiments are exemplarily described with reference to the corresponding figures in the accompanying drawings, and the descriptions are not to be construed as limiting the embodiments. Elements in the accompanying drawings that have same reference numerals are represented as similar elements, and unless otherwise particularly stated, the figures in the accompanying drawings are not drawn to scale.
FIG. 1 is a schematic diagram of a structure of an aerosol generating apparatus according to an embodiment of this application;
FIG. 2 is a cross-sectional view of an atomizer according to an embodiment of this application;
FIG. 3 is an exploded view of an atomizer according to an embodiment of this application;
FIG. 4 is a perspective view of a ceramic heating body from one perspective according to an embodiment of this application;
FIG. 5 is a perspective view of a ceramic heating body from another perspective according to an embodiment of this application;
FIG. 6 is a cross-sectional view of a ceramic heating body according to an embodiment of this application;
FIG. 7 is an exploded view of an atomization assembly according to an embodiment of this application;
FIG. 8 is a perspective view of a third sealing element according to an embodiment of this application; and
FIG. 9 is a perspective view of a first sealing element according to an embodiment of this application.

In the accompanying drawings:
100. Atomizer; 200. power supply assembly;
10. housing assembly; 11. suction nozzle; 12. shell; 13. bottom cap; 14. sealing sleeve; 15. engagement member; 110. suction nozzle opening; 121. liquid storage cavity; 131. threaded sleeve; 132. air inlet; 133. electrical connecting member;
20. atomization assembly; 21. porous liquid guide element; 22. heating element; 23. bracket; 25. atomization cavity; 26. third sealing element; 27. ceramic heating body; 211. first section; 212. second section; 213. step surface; 215. groove; 216. convex point; 221. conductive pin; 231. hole; 232. liquid guide cavity; 233. vent tube; 234. second notch; 235. accommodating cavity; 241. second sealing element; 242. first sealing element; 243. first notch; 244. fitting groove; 261. cavity; 262. vent hole; 263. air guide tube; 264. baffle plate; 265. lead post; 266. convex edge; 2151. first groove segment; 2152. second groove segment; 2153. first groove; and 2154. second groove.

### DESCRIPTION OF EMBODIMENTS

For ease of understanding of this application, this application is described in further detail below with reference to the accompanying drawings and specific implementations.

It should be noted that, all directional indications (for example, up, down, left, right, front, back, horizontal, and vertical) in embodiments of this application are only used for explaining relative position relationships, movement situations, or the like between the various components in a specific posture (as shown in the accompanying drawings). If the specific posture changes, the directional indications change accordingly. The "connection" may be a direct connection or an indirect connection, and the "arrange" and "being arranged on" may be directly arranged on or indirectly arranged on.

In addition, descriptions such as "first" and "second" in this application are merely intended for a purpose of description, and shall not be understood as an indication or implication of relative importance or implicit indication of a quantity of indicated technical features. Therefore, features defining "first" and "second" can explicitly or implicitly include at least one of the features.

An aerosol generating apparatus includes an atomizer 100 and a power supply assembly 200. The power supply assembly 200 provides electric drive for the atomizer 100. The aerosol generating apparatus generates an aerosol by atomizing a liquid substrate stored in the aerosol generating apparatus. According to different liquid substrates stored in the aerosol generating apparatus, the aerosol generating apparatus may be grouped into an e-cigarette and a medical device. The liquid substrate stored inside the e-cigarette includes nicotine preparations, glycerin, propylene glycol, flavors, and fragrances, flavor components, and the like. An aerosol generated by inhaling the e-cigarette by a user mainly meets a requirement for the nicotine or the flavor components. As the medical device, the liquid substrate stored inside medical device includes active functional components, glycerol, propylene glycol, and the like. An aerosol generated by inhaling the device by a user is mainly used to treat respiratory diseases, or to inhale specific medicinal active ingredients through the lung. Related implementations provided in this application may be applied to the foregoing two types of devices, and are not limited herein.

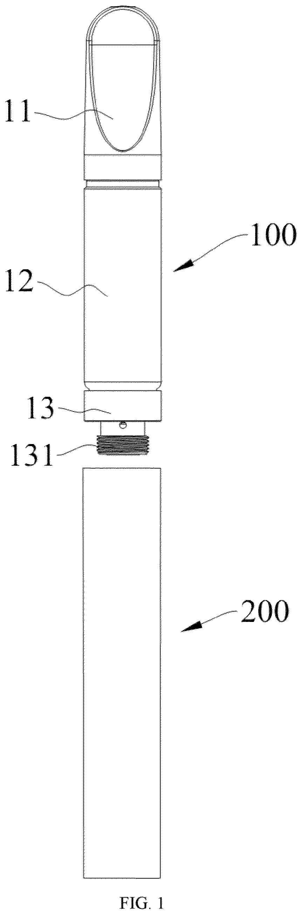

The atomizer 100 and the power supply assembly 200 may be accommodated inside one housing, to form a disposable aerosol generating apparatus that is small in size and easy to carry. The atomizer 100 and the power supply assembly 200 may also be configured as two independent assemblies, and the two assemblies are connected in a detachable connection manner, to form a combined aerosol generating apparatus. In one example, an accommodating cavity is defined and formed in a part of an inner cavity of the housing of the power supply assembly 200. The atomizer 100 may be inserted from an end of the housing of the power supply assembly 200 and at least a part of a surface of the atomizer 100 may be kept inside the accommodating cavity. A stable connection may be formed between the two assemblies in a manner of magnetic adsorption or buckle connection. In another example, as shown in FIG. 1, a threaded sleeve 131 is arranged at an end portion of the atomizer 100, a threaded groove is provided at an end portion of the power supply assembly 200, and the two assemblies are in threaded connection with each other. Electrical connection assemblies are arranged at a connection end portion of the atomizer 100 and a connection end portion of the power supply assembly 200, so that after the two assemblies are connected, a circuit between the two assemblies is connected. An internal structure of the power supply assembly 200 may be configured as a common form in the conventional technology. For example, a control module and a charging module are arranged inside the power supply assembly 200. This is not described in detail in the implementation part of this application.

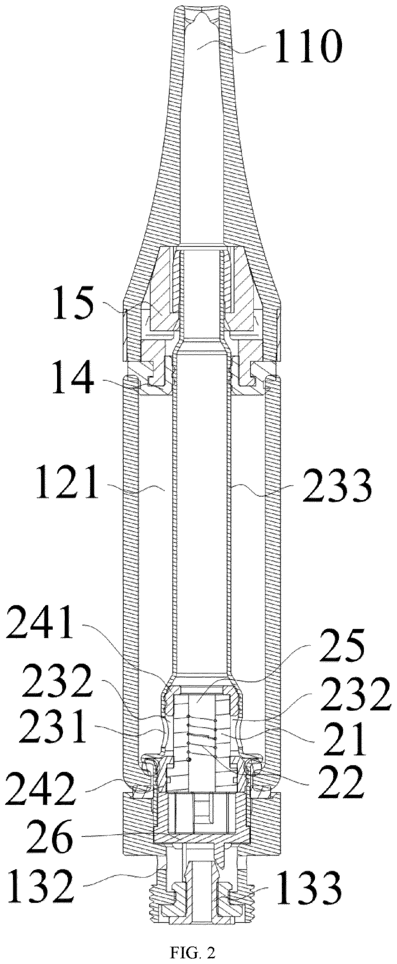

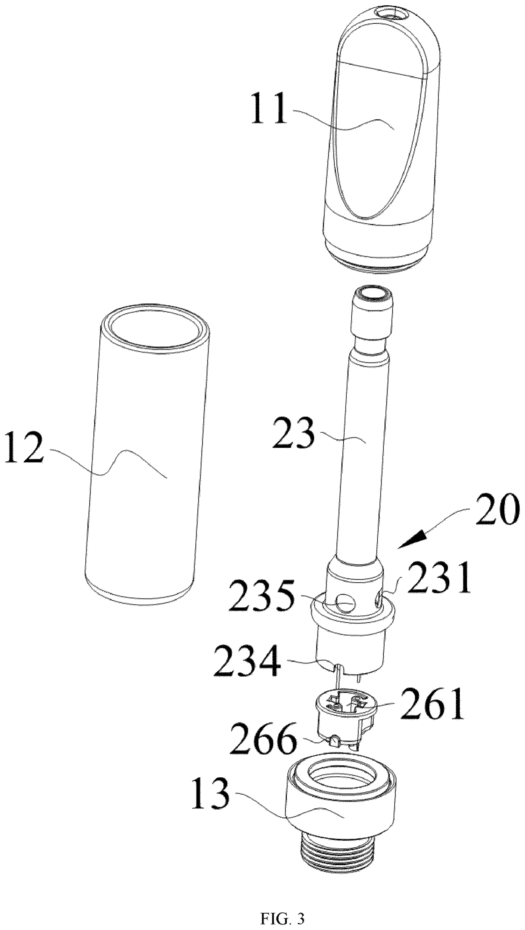

The following part mainly describes in detail an internal structure of an assembly of the atomizer 100. Referring to FIG. 2 and FIG. 3, a structure of the atomizer 100 that is substantially in a shape of a cylinder is used as an example. It should be noted that, the atomizer 100 may be of any other shape in the conventional technology, which does not affect implementation of implementations provided in this application. The atomizer 100 includes a housing assembly 10. Viewed from the outside, the housing assembly 10 includes a suction nozzle 11 and a shell 12. The suction nozzle 11 is substantially flat. When using the aerosol generating apparatus, a user is mainly in contact with the suction nozzle 11. Therefore, the suction nozzle 11 may preferably be made of a plastic material with higher safety performance, such as PP (polypropylene). The shell 12 may preferably be made of a transparent or translucent material, such as glass, so that the user can observe consumption of a liquid substrate inside the shell 12. The suction nozzle 11 is connected to one end of the shell 12, and a bottom cap 13 is connected to the other end of the shell 12. A threaded sleeve 131 is arranged on an end of the bottom cap 13.

An atomization assembly 20 is mainly mounted inside the housing assembly 10 of the atomizer 100. The atomization assembly 20 is substantially in a shape of a hollow tubular structure. A liquid storage cavity 121 is defined and formed in a part of a space of an inner cavity of the shell 12. The liquid storage cavity 121 is configured to be provided surrounding the atomization assembly 20, so that a liquid substrate inside the liquid storage cavity 121 enters inside of the atomization assembly 20 at a uniform rate.

As a core functional assembly of the atomizer 100, the atomization assembly 20 mainly includes a liquid guide element and a heating element 22. The liquid substrate stored in the liquid storage cavity 121 may flow to the liquid guide element and be absorbed and stored by the liquid guide element. The heating element 22 is configured to be combined with at least a part of the liquid guide element, and atomize the liquid substrate transferred by the liquid guide element to generate an aerosol. The heating element 22 may be a spiral heating wire made of at least one of materials such as stainless steel, nickel-chromium alloy, iron-chromium-aluminum alloy, and metal titanium, or a heating sheet with a grid structure. The liquid guide element may be substantially divided into two categories according to different preparation materials. A first type of liquid guide element is made of materials with a capillary structure, such as non-woven fabrics and fiber cotton. In addition to being capable of transferring the liquid substrate, the type of liquid guide element may also store a specific amount of liquid substrate. A second type of liquid guide element is made of ceramic materials with a hard porous structure. The type of liquid guide element has sufficient hardness, to provide support for the heating element 22. In the conventional technology, the foregoing two types of liquid guide elements are usually arranged in combination to form a liquid guide assembly, so that a hard part of the ceramic liquid guide element is configured to fix the heating element 22, and a soft part of the liquid guide cotton is arranged surrounding the ceramic liquid guide element to improve a liquid guide capacity. In addition, the soft liquid guide cotton is wound surrounding an outer surface of the ceramic liquid guide element, which may effectively seal the ceramic liquid guide element, and prevent the liquid substrate from leaking along a surface of the ceramic liquid guide element. However, an assembly process of the liquid guide assembly that is arranged in combination is complex. In particular, the liquid guide cotton needs to be manually wound and fixed on an outer periphery of the ceramic liquid guide element, which limits the atomization assembly 20 to be automatically assembled. In addition, there are great differences in winding and fixing operation methods of different operators and arrangement positions of the liquid guide cotton, which leads to differences in the liquid guide capacity and an atomization effect of the aerosol generating apparatus.

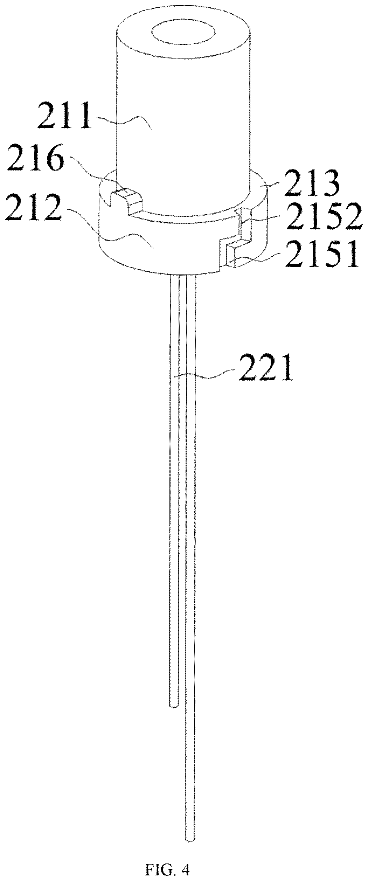

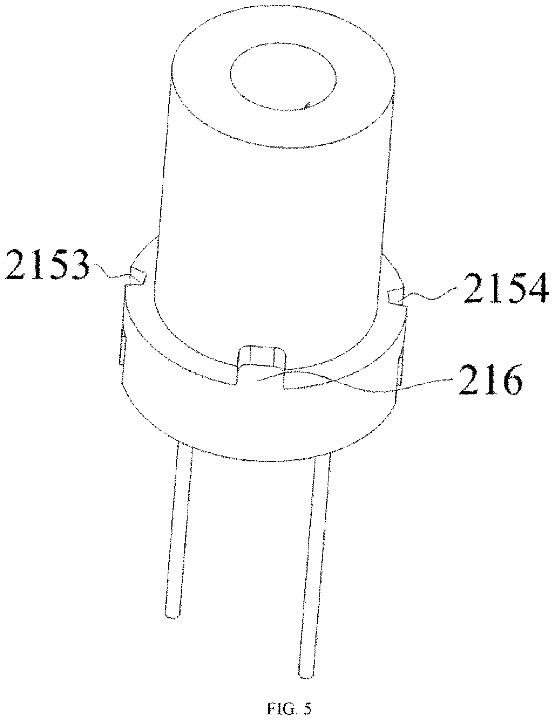

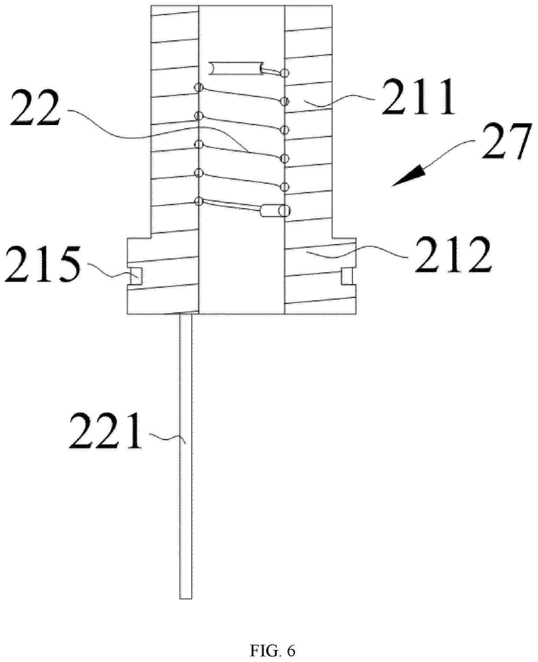

To optimize a structure of the liquid guide element and sealing performance of the atomizer 100, an embodiment of this application provides a porous liquid guide element 21 with an excellent structure and excellent performance. Referring to FIG. 4 to FIG. 6, the porous liquid guide element 21 is made of a ceramic material, and is configured to include a hollow tubular structure. The porous liquid guide element 21 may be substantially divided into two sections, namely, a first section 211 and a second section 212 separately, in an axial direction of the porous liquid guide element 21. An outer diameter of the first section 211 is less than an outer diameter of the second section 212, and a step surface 213 is thereby formed between the second section 212 and the first section 211. In some examples, an inner diameter of the first section 211 of the porous liquid guide element 21 is inconsistent with an inner diameter of the second section 212, and an inner cavity of the porous liquid guide element 21 is configured as an irregular cavity 261. Therefore, a wall thickness of the first section 211 and a wall thickness of the second section 212 are optimized according to a shape design of the cavity 261. In a preferred implementation, the wall thickness of the first section 211 is less than the wall thickness of the second section 212. The first section 211 of the porous liquid guide element 21 is configured to transfer and store a part of a liquid substrate, and the second section 212 of the porous liquid guide element 21 has a greater wall thickness and is mainly configured to store the liquid substrate. Specifically, the wall thickness of the first section 211 is set to range from 0.5 mm to 1.2 mm. A specific wall thickness of the first section 211 of the porous liquid guide element 21 may be set to any value between 0.5 and 1.2, such as 0.7 mm, 0.9 mm, 1.0 mm, and 1.2 mm. The wall thickness of the second section 212 may be set to range from 1.2 mm to 3 mm. A specific wall thickness of the second section 212 of the porous liquid guide element 21 may be set to any value between 1.2 and 3, such as 1.5 mm, 1.8 mm, 2.0 mm, and 2.5 mm. In one example, the wall thickness of the first section 211 of the porous liquid guide element 21 is 1 mm, and the wall thickness of the second section 212 of the porous liquid guide element 21 is 1.5 mm. The heating element 22 is fixed in the inner cavity of the porous liquid guide element 21. The porous liquid guide element 21 transfers the liquid substrate absorbed by the porous liquid guide element 21 to the heating element 22, and the heating element 22 atomizes the liquid substrate to generate an aerosol. For the tubular porous liquid guide element 21, the liquid substrate enters the tubular porous liquid guide element 21 through an outer surface of the porous liquid guide element 21, and penetrates into the inside of the porous liquid guide element 21. The porous liquid guide element 21 mainly absorbs and transfers the liquid substrate through an internal porous structure of the porous liquid guide element 21. Relying on a capillary force, the liquid substrate penetrates into the inside of the porous liquid guide element 21 layer by layer, and finally reaches an inner surface of the porous liquid guide element 21, to be transferred to a surface of the heating element 22. Therefore, the thicker the wall thickness of the porous liquid guide element 21, the more liquid substrate the porous liquid guide element 21 can store, and the greater the liquid-preserving capacity of the porous liquid guide element 21. In addition, the thicker the wall thickness of the porous liquid guide element 21, the larger the area of an end surface of the porous liquid guide element 21 facing away from the liquid storage cavity 121, and the greater the tension of liquid when the liquid adheres to the end surface of the porous liquid guide element 21. Because the liquid is difficult to escape from the end surface of the porous liquid guide element 21, a possibility of liquid leakage is low. The thicker the wall thickness of the porous liquid guide element 21, the greater the mass transfer resistance of the liquid substrate when being transferred from the outer surface of the porous liquid guide element 21 to the inner surface of the porous liquid guide element 21, resulting in a lower liquid guide capacity of the porous liquid guide element 21. After repeated tests and analysis on liquid guide rates of liquid substrates with a plurality of properties and heating efficiency of the heating element 22, and considering a factor of a design size of the atomizer 100 product, when the wall thickness of the porous liquid guide element 21 ranges from 0.5 mm to 1.2 mm, a liquid guide rate of the porous liquid guide element 21 and a heating rate of the heating element 22 may maintain a good balance. In other words, the heating element 22 may atomize, in a timely manner, the liquid substrate transferred by the porous liquid guide element 21. A case in which the liquid substrate accumulates near the heating element 22 and boil, and emit a gurgling sound, or the liquid substrate is not supplied in a timely manner, causing the heating element 22 to dry burn in the absence of the liquid substrate does not occur. In addition, in a preferred implementation, the wall thickness of the first section 211 of the porous liquid guide element 21 is uniform. Therefore, a liquid guide capacity and a liquid storage capacity of the first section 211 of the porous liquid guide element 21 are uniform. Further, the heating element 22 may atomize, at a uniform rate, the transferred liquid substrate, so that the atomizer 100 may keep a stable TPM (amount of vapor inhaled per puff).

By arranging two sections of porous liquid guide element 21 with different wall thicknesses and fixing the heating element 22 in an inner cavity of the first section 211 of the porous liquid guide element 21, the inner cavity of the porous liquid guide element 21 is mainly configured to provide a channel for an airflow. The wall thickness of the second section 212 of the porous liquid guide element 21 is great, and the second section 212 of the porous liquid guide element 21 has a strong storage capacity for the liquid substrate, so that an area of the porous liquid guide element 21 away from the heating element 22 has a strong liquid storage capacity and may prevent the liquid substrate from leaking from a bottom end portion of the porous liquid guide element 21. The wall thickness of the second section 212 of the porous liquid guide element 21 is greater than the wall thickness of the first section 211. A step surface 213 is formed between the first section 211 and the second section 212, so that the porous liquid guide element 21 may be fixed to a support assembly of the porous liquid guide element 21. After testing and analysis and calculation are performed on liquid substrates with different viscosities, when the wall thickness of the porous liquid guide element 21 is greater than 1.2 mm, sufficient mass transfer resistance may be formed inside the porous liquid guide element 21, so that the liquid substrate may be stored inside the porous liquid guide element 21. A case in which the excessive liquid substrate cannot be stored by the porous liquid guide element 21, and the liquid substrate is away from the heating element 22, and cannot be atomized in a timely manner, resulting in leakage of the liquid substrate does not occur. In addition, there is an integral structure between the first section 211 and the second section 212 of the porous liquid guide element 21. Therefore, there is a capillary force between the first section 211 and the second section 212. The liquid substrate stored in the first section 211 of the porous liquid guide element 21 and the liquid substrate stored in the second section 212 may flow with each other. When there are a lot of liquid substrates stored in a unit volume area of the first section 211 of the porous liquid guide element 21, the liquid substrate may flow to an area of the second section 212 of the porous liquid guide element 21. When there are few liquid substrates stored in a unit volume area of the first section 211 of the porous liquid guide element 21, the liquid substrate may flow from the area of the second section 212 of the porous liquid guide element 21 to the area of the first section 211 of the porous liquid guide element 21. Considering an overall volume and a weight of the porous liquid guide element 21, a maximum value of the wall thickness of the second section 212 of the porous liquid guide element 21 is 3 mm. It may be understood that, if the wall thickness of the second section 212 of the porous liquid guide element 21 is too great, an overall weight of the porous liquid guide element 21 increases, making it more difficult to fix the porous liquid guide element 21 inside the atomizer 100, and an entire volume occupied by the porous liquid guide element 21 increases, which is not conducive to optimization of an overall structure of the atomizer 100.

In one example provided in this application, the heating element 22 is arranged in the form of a heating wire. The heating wire is uniformly wound and fixed on an inner wall of the porous liquid guide element 21, and is spirally distributed. Winding spacings of the heating wire are basically the same, to balance overall heating efficiency of the heating element 22. The heating wire is made of a material with an excellent high resistance property, such as iron-chromium-aluminum alloy or nickel-chromium alloy. Conductive pins 221 are connected to two ends of the heating wire, which are separately a positive conductive pin and a negative conductive pin. The heating element 22 is electrically connected to an electrical connecting member on the atomizer 100 through the conductive pins, to connect the heating element 22 to a power supply on the power supply assembly 200. The conductive pins 221 are made of a material with a low resistance property, such as nickel. The heating wire is combined with the porous liquid guide element 21 through an injection molding process, and the heating wire is basically arranged on an inner wall of the first section 211 of the porous liquid guide element 21, and the two conductive pins on the two ends of the heating wire extend inside the porous liquid guide element 21, pass through the second section 212 of the porous liquid guide element 21, and extend out from a bottom end surface of the second section 212 of the porous liquid guide element 21.

The conductive pins of the heating element 22 also generate heat in a conduction process, and the heat does not produce effective value in an actual heating process. Therefore, to avoid the two conductive pins from generating much heat and causing energy waste, in a preferred implementation provided in this application, a wire diameter of the heating wire and a wire diameter of each of the conductive pins need to be controlled in a proper range. According to a principle of resistance heating and repeated analysis and calculation, a wire diameter of the heating element 22 ranges from 0.14 mm to 0.18 mm. If the heating element 22 is made of the iron-chromium-aluminum alloy material, a resistance value of the corresponding heating element 22 ranges from 1.0 S2 to 1.6 Q. A specific wire diameter of the heating element 22 may be optimally designed according to a heating power of the heating element needed by the atomizer 100, and the wire diameter may be any value between 0.14 mm to 0.18 mm. A wire diameter of each of the conductive pins on the two ends of the heating element 22 ranges from 0.24 mm to 0.30 mm, which may be adjusted according to the specific wire diameter of the heating element, so that any value between 0.24 mm to 0.30 mm may be selected.

Further, the heating element 22 extends substantially in a longitudinal direction of the porous liquid guide element 21, and a longitudinal extension range of the heating element 22 does not exceed a longitudinal extension range of the first section of the porous liquid guide element 21. Therefore, the second section 212 of the porous liquid guide element 21 is configured as a liquid guide area. To prevent heat from being transferred to the outside through the second section 212 of the porous liquid guide element 21, the wall thickness of the second section 212 of the porous liquid guide element 21 is thicken, which is conducive to increasing a heat capacity of the second section 212, so that the heat may be more effectively kept on the second section 212 of the porous liquid guide element 21. This prevents diffusion of excessive heat to a connecting assembly around the second section 212 of the porous liquid guide element 21, which is conducive to improving utilization efficiency of the heat generated by the heating element 22.

In one embodiment of this application, a ceramic heating body 27 is further provided. The ceramic heating body 27 includes the porous liquid guide element 21 and the heating element 22. The porous liquid guide element 21 is configured to transfer the liquid substrate to the heating element 22. Further, embodiments of this application further provide a method for preparing the ceramic heating body 27, which includes the following steps.
1. Preparation of a slurry: Mix ceramic powder with sintering agent, organic additives, and pore-forming agent to prepare a ceramic slurry. The ceramic powder includes at least one of alumina, zirconia, silicon oxide, silicon nitride, silicon carbide, cordierite, or mullite; the sintering agent includes at least one of calcium carbonate, magnesium oxide, lanthanum oxide, barium oxide, calcium oxide, and lithium oxide; and the pore-forming agent includes at least one of mineral wax, white wax, beeswax, and ozokerite. The organic additives are mainly dispersants, which include at least one of fatty acid dispersants and acrylic resin dispersants. By adding the organic additives to the mixed slurry, uniformity of mixing the ceramic slurry may be enhanced. Further, based on a weight percentage of the ceramic slurry, a mass percentage of the ceramic powder ranges from 30% to 50%, a mass percentage of the sintering agent ranges from 15% to 30%, and a mass percentage of the pore-forming agent ranges from 20% to 40%.
2. Obtain a ceramic green body through injection molding by using a ceramic injection molding machine, where an injection pressure in an injection molding process ranges from 0.5 MPa to 5 MPa, and a temperature ranges from 50°C to 100°C. In the ceramic injection molding process, the heating element 22 is fixed in a mold cavity, so that the heating element 22 and the porous liquid guide element 21 are formed together.
3. Degrease and sinter the ceramic green body to obtain a porous ceramic.

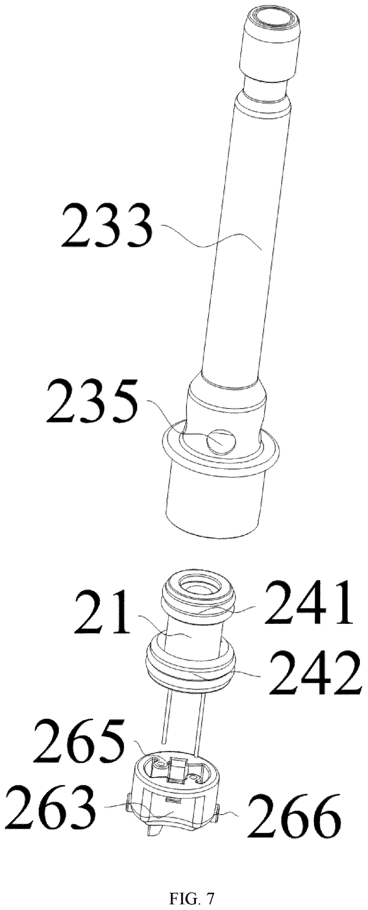

The atomization assembly 20 further includes a bracket 23. Referring to FIG. 2 and FIG. 7, the porous liquid guide element 21 is configured as a substantially hollow cylindrical structure, which is fixed in an inner cavity of the substantially tubular bracket 23. The bracket 23 is preferably formed by stretching a metal material, and includes an accommodating cavity 235. The porous liquid guide element 21 is accommodated inside the accommodating cavity 235. The accommodating cavity 235 of the bracket 23 includes a first part and a second part with different inner diameters. The first part has a small inner diameter, and is configured to accommodate the first section 211 of the porous liquid guide element 21. The second part has a large inner diameter, and is configured to accommodate the second section 212 of the porous liquid guide element 21. A liquid storage cavity 121 is defined and formed between an outer wall surface of the bracket 23 and an inner wall surface of the shell 12. A hole 231 is further provided on the bracket 23, and a liquid substrate inside the liquid storage cavity 121 may directly enter the inside of the accommodating cavity 235 through the hole 231.

To facilitate the liquid substrate to be introduced into the porous liquid guide element 21 more quickly, in addition to accommodating the porous liquid guide element 21, an extra space is further arranged inside the accommodating cavity 235. In other words, a substantially annular liquid guide cavity 232 is defined between an inner wall of the bracket 23 and an outer surface of the porous liquid guide element 21 that is not covered by a first sealing element 242 and a second sealing element 241. The liquid guide cavity 232 is configured to further store the liquid substrate transferred through the liquid storage cavity 121. In addition, because the liquid guide cavity 232 is provided, liquid guide cotton does not need to be wound around a periphery of the porous liquid guide element 21. A manual operation of cotton winding is eliminated, and the atomization assembly 20 may be automatically assembled. The porous liquid guide element 21 is in a structure of being surrounded by the liquid guide cavity 232. Therefore, the liquid substrate is transferred to the porous liquid guide element 21 mainly by a capillary force of the porous liquid guide element 21, and a liquid guide rate is faster. In addition, liquid and mass inside the liquid storage cavity 121 enter the liquid guide cavity 232 only relying on a liquid level, rather than a capillary force of the liquid guide cotton. Therefore, the porous liquid guide element 21 is in a stable liquid supply environment, which is more conducive to balancing the liquid guide rate of the porous liquid guide element 21, and is conducive to stabilizing a TPM (amount of vapor inhaled per puff) of the atomizer 100.

Further, the porous liquid guide element 21 is configured as a two-section structure, and the heating element 22 is fixed on the inner wall of the first section 211 of the porous liquid guide element 21. The liquid guide cavity 232 is provided on a periphery of the first section 211 of the porous liquid guide element 21, and the liquid substrate may be quickly transferred on the first section 211 of the porous liquid guide element 21. As a support section of the porous liquid guide element 21, the second section 212 of the porous liquid guide element 21 has a strong liquid storage capacity, and the liquid substrate may be stored inside the porous liquid guide element 21, and is not leaked through an end portion of the porous liquid guide element 21. Specifically, the second section 212 of the porous liquid guide element 21 forms a step surface 213 relative to the first section 211 of the porous liquid guide element 21, and the porous liquid guide element 21 is fixed inside the bracket 23 through the step surface 213. To prevent leakage of the liquid substrate, two ends of the porous liquid guide element 21 are fixed inside the bracket 23 through two sealing elements, and two ends of the liquid guide cavity 231 are sealed by the sealing elements arranged on the two ends of the porous liquid guide element 21. Specifically, because the second section 212 of the porous liquid guide element 21 is at a lower end of a liquid level in the liquid storage cavity 121, the liquid substrate mainly leaks to the outside through a connection gap of the second section 212 of the porous liquid guide element 21. Therefore, the first sealing element 242 that is arranged on an outer surface of the second section 212 of the porous liquid guide element 21 mainly seals the porous liquid guide element 21. The first sealing element 242 is made of a silicone material, which has a better sealing effect, and the first sealing element 242 includes a sleeve portion and a radial extension portion. The sleeve portion is arranged surrounding an outer side surface of the second section 212 of the porous liquid guide element 21, and the radial extension portion is arranged covering the step surface 213. The step surface 213 of the porous liquid guide element 21 abuts against an inner wall surface of the first sealing element 242 in a sealing manner, making it difficult for the liquid substrate to leak to the outside through a connection end of the porous liquid guide element 21. Further, the first sealing element 242 is substantially configured as a sleeve-shaped structure, and is sleeved on the entire outer side surface of the second section 212 of the porous liquid guide element 21 and the step surface 213. Several convex ribs are further arranged on the outer surface of the first sealing element 242, so that the first sealing element 242 may firmly seal the connection gap between the bottom end of the second section 212 of the porous liquid guide element 21 and the inner wall of the bracket 23, making it difficult for the liquid substrate to leak to the outside through the joint.

Further, the first sealing element 242 is also arranged surrounding an upper end of the first section 211 of the porous liquid guide element 21. In addition to being made of the silicone material, the second sealing element 241 may be further made of fiber cotton for sealing. The second sealing element 241 is substantially configured as the sleeve-shaped structure, and is sleeved on a part of the outer surface of the first section 211 of the porous liquid guide element 21. Specifically, the second sealing element 241 covers an upper side surface of the first section 211 of the liquid guide element 21 and a part of a top end surface of the first section 211 of the liquid guide element 21, and a top end surface of the second sealing element 241 abuts against the inner wall of the bracket 23 in a longitudinal direction. Several convex ribs are further arranged on the outer surface of the second sealing element 241, so that the second sealing element 241 may firmly seal a connection gap between a top end of the first section 211 of the porous liquid guide element 21 and the inner wall of the bracket 23, making it difficult for the liquid substrate to leak to the outside through the joint. In a preferred implementation, the longitudinal extension range of the heating element 22 is not in a longitudinal extension range of the second sealing element 241, so that the heat generated by the heating element 22 may be less transmitted to the second sealing element 241 and the bracket 23, resulting in less heat loss.

An atomization cavity 25 is defined and formed in the accommodating cavity 235 of the bracket 23, and an air inlet channel and an air outlet channel that are in communication with the atomization cavity 25 are further provided inside the shell 12. In one example, the bracket 23 extends in a longitudinal direction in an axial direction of the bracket 23 to form an air outlet tube 233. An end of the air outlet tube 233 extends into an inner cavity of the suction nozzle 11, and is in buckle connection to the suction nozzle 11. An aerosol generated in the atomization cavity 25 may be guided into a suction nozzle opening 110 through an inner cavity of the air outlet tube 233, so that the aerosol is inhaled by a user. In an optional implementation, the air outlet tube 233 may also be separately formed, and is connected to an end of the bracket 23. In a preferred implementation, a tight buckle connection structure is configured between the air outlet tube 233 and the suction nozzle 11. Specifically, a buckle is arranged on an outer wall of the air outlet tube 233, and an engagement member 15 is arranged in the inner cavity of the suction nozzle 11. The engagement member 15 is substantially in a shape of a plunger, and several undercut structures that are spaced apart are arranged on the engagement member 15. The undercut structures span a side surface of the buckle on the air outlet tube 233 and abut against a bottom end surface of the buckle in a longitudinal direction. Further, an upper sealing sleeve 14 is arranged between the suction nozzle 11 and the shell 12, and an annular groove is provided on the upper sealing sleeve 14. A lower end of the engagement member 15 is accommodated in an annular groove of the upper sealing sleeve 14, and an inner wall surface of the upper sealing sleeve 14 abuts against an outer wall surface of the air outlet tube 233.

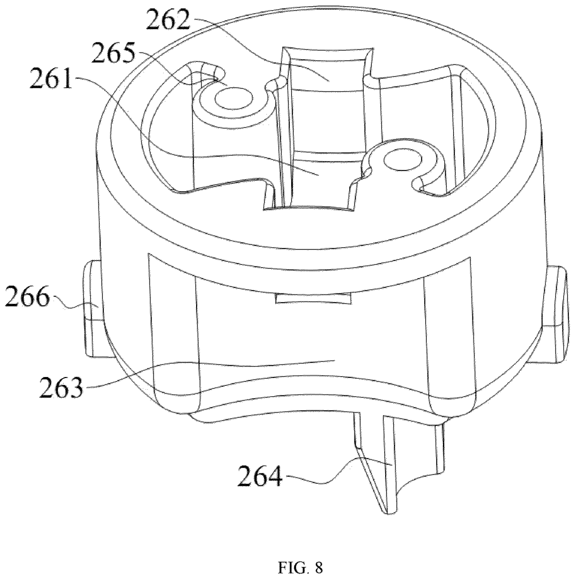

A third sealing element 26 is further arranged at an end of the bracket 23 facing away from the air outlet channel. Referring to FIG. 2, FIG. 7, and FIG. 8, the third sealing element 26 abuts against the first sealing element 242 and a lower end surface of the second section 212 of the porous liquid guide element 21 in a longitudinal direction. The third sealing element 26 is substantially configured as an irregular plug, which includes a bottom wall and a side wall. The bottom wall and the side wall are enclosed to form an open cavity 261. The cavity 261 is provided facing the atomization cavity 25. A part of the side wall of the third sealing element 26 abuts against the inner wall surface of the bracket 23, and a part of the wall surface is concave. An inner concave wall surface of the third sealing element 26 and the inner wall surface of the bracket 23 define and form an air guide cavity 263. A vent hole 262 is further provided on the side wall of the third sealing element 26. The air guide cavity 263 is in communication with the vent hole 262. A bottom cap 13 is arranged on a lower end of the shell 12. Amain body portion of the third sealing element 26 is accommodated in the inner cavity of the bracket 23. A lower end surface of the third sealing element 26 abuts against the bottom cap 13. There is an air inlet 132 on the bottom cap 13, to guide an external airflow into an inner cavity of the shell 12. Two air inlets 132 are provided on two sides of the bottom cap 13. Two vent holes 262 are also provided on opposite side walls of the third sealing element 26. Correspondingly, two air guide cavities 263 are defined and formed on two sides of the third sealing element 26. The external airflow enters the air guide cavities 263 through the air inlets 132 on two sides, further, enters the vent holes 262, enters the inner cavity of the bracket 23 through the cavity 261 of the third sealing element 26, and then is introduced into the atomization cavity 25. Because the cavity 261 of the third sealing element 26 is provided facing the atomization cavity 25, the cavity 261 of the third sealing element 26 may receive condensate generated inside the atomization cavity 25, thereby preventing the condensate from leaking to the outside.

As a core unit assembly of the atomizer 100, the atomization assembly 20 is configured to be replaceable. By removing the bottom cap 13 on the lower end of the shell 12, the user may remove the atomization assembly 20 for replacement, which is conducive to extending an overall service life of the atomizer 100.

A pair of electrical connecting members 133 are arranged on the bottom cap 13. The electrical connecting member 133 is configured in the form of an electrode ring. The electrode ring includes an inner electrode and an outer electrode that are spaced apart. The inner electrode is connected to the positive conductive pin that is connected to the heating element 22, and the outer electrode is connected to the negative conductive pin that is connected to the heating element 22. The positive conductive pin and the negative conductive pin that are connected to the two ends of the heating element 22 need to separately pass through the third sealing element 26 to be connected to the inner electrode and the outer electrode of the electrode ring.

Further, a baffle plate 264 is further arranged at a bottom end of the third sealing element 26. The baffle plate 264 extends from a bottom end surface of the third sealing element 26 toward the inner electrode of the electrical connecting member 133. Apart of a wall surface of the baffle plate 264 may be further arranged close to the inner electrode. The baffle plate 264 has functions in many aspects. Because only one insulating ring is arranged between the inner electrode and the outer electrode, a distance is close, and the inner electrode is arranged protruding relative to the outer electrode, without blocking, the negative conductive pin is easy to be in contact with the inner electrode, causing a short circuit. The arrangement of the baffle plate 264 prevents the negative conductive pin from contacting the inner electrode. Instead, the negative conductive pin can only be attached to the wall surface of the baffle plate 264, thereby avoiding occurrence of the short circuit. In addition, the baffle plate 264 may further play a liquid guide role. When a part of liquid overflows from the vent hole 262 of the third sealing element 26, the liquid is guided to flow to a peripheral area of the inner electrode due to the action of the baffle plate 264, so that no liquid flows into the inner electrode to corrode an electrode assembly. Further, internally hollow lead posts 265 are arranged on the third sealing element 26. The conductive pins 221 connected to the two ends of the heating element 22 extend out from the bottom end surface of the porous liquid guide element 21, extend out from the two lead posts 265 arranged on the third sealing element 26, and then are electrically connected to the electrode ring. In a preferred implementation, top ends of the two lead posts 265 are in contact with the bottom end surface of the porous liquid guide element 21, so that the third sealing element 26 further provides support in a longitudinal direction for the porous liquid guide element 21.

In still another embodiment provided in this application, a vent channel is provided between the porous liquid guide element 21 and the first sealing element 242 and/or the second sealing element 241. An airflow enters the liquid storage cavity 121 through the vent channel to prevent negative pressure from being generated inside the liquid storage cavity 121 and causing poor liquid guide. In one example, a second vent channel that is in communication with the air outlet tube is provided at a connection surface between the second sealing element 241 and the porous liquid guide element 21, or a second vent channel that is in communication with the air outlet tube is provided on the second sealing element 241.

Further, a first vent channel is provided between the second section 212 of the porous liquid guide element 21 and the first sealing element 242. The first vent channel may be provided on the first sealing element 242, and the first vent channel may also be provided at the connection gap between the first sealing element 242 and the second section 212 of the porous liquid guide element 21. In a preferred implementation, the first vent channel includes a groove 215 provided on the outer wall surface of the porous liquid guide element 21, and the groove 215 may guide the airflow to the inside of the liquid storage cavity 121. Specifically, the groove 215 is provided on the second section 212 of the porous liquid guide element 21, and extends from the bottom end surface of the second section 212 of the porous liquid guide element 21 to a top end surface of the second section 212, where the top end surface of the porous liquid guide element 21 is arranged close to the liquid storage cavity 121. An inlet of the groove 215 is in communication with an air inlet channel at a bottom portion of the bracket 23. In one example provided in this application, the inlet of the groove 215 is in communication with the cavity 261 of the third sealing element 26, so that the airflow may enter the groove 215. An outlet of the groove 215 is in communication with the liquid guide cavity 232, and the airflow may enter through the groove on the porous liquid guide element 21, enter the liquid guide cavity 232 through the groove 215, and then enter the liquid storage cavity 121. Setting of a specific position of the groove 215 is mainly determined by a sealing and fixing structure of a lower end of the porous liquid guide element 21. A main purpose is to ensure that the outlet of the groove 215 in communication with the liquid storage cavity 121, and in addition, provision of the groove 215 cannot cause leakage of the liquid. If the first sealing element 242 not only covers the outer surface of the second section 212 of the porous liquid guide element 21, but also covers a part of a surface of the first section 211 of the porous liquid guide element 21, a part of a section of the groove 215 is provided on the first section 211 of the porous liquid guide element 21, and a part of a section of the groove 215 is provided on the second section 212 of the porous liquid guide element 21. In one example provided in this application, the lower end of the porous liquid guide element 21 mainly relies on the step surface 213 of the second section 212. The first sealing element 242 basically covers a side surface of the second section 212 of the porous liquid guide element 21 and the step surface 213. Therefore, the groove 215 extends from the bottom end surface of the second section 212 of the porous liquid guide element 21 to the step surface 213 of the second section 212, where the step surface 213 is arranged close to the liquid storage cavity 121. In a preferred implementation, to facilitate the airflow to be introduced into the inside of the liquid storage cavity 121 as quickly as possible, the outlet of the groove 215 is provided close to the hole 231 on the bracket 23, so that the airflow enters the liquid storage cavity 121 through the hole 231 after flowing out of the groove 215. When the first sealing element 242 completely covers the top end surface of the porous liquid guide element 21, a first notch 243 is further provided on the first sealing element 242, and the first notch 243 is in communication with the groove 215 and the liquid storage cavity 121. If the first sealing element 242 covers only a part of the top end surface of the porous liquid guide element 21, and the outlet of the groove 215 may be in communication with the liquid guide cavity 121, the first notch 243 does not need to be provided on the first sealing element 242. To prevent the liquid from leaking along the groove 215, the groove 215 is provided as a meandering extension structure. In other words, the outlet of the groove 215 and the inlet of the groove 215 are not in a straight line. The liquid may only flow in a meandering manner after entering from the outlet of the groove 215, and a part of a wall surface of the groove 215 hinders further flow of the liquid substrate. In one example, the groove 215 extends in a substantially Z shape, or at least one of an S shape, a spiral shape, a zigzag shape, and a labyrinth shape. In another example, the groove 215 includes a first groove segment 2151 and a second groove segment 2152. The first groove segment 2151 is in communication with the air inlet channel, the second groove segment 2152 is in communication with the liquid storage cavity 121, and a width of the first groove segment 2151 is less than a width of the second groove segment 2152. This allows the airflow to quickly enter the liquid storage cavity 121 from the wide outlet of the second groove segment 2152, and in addition, it makes it difficult for the liquid to flow out through the narrow outlet of the first groove segment 2151.

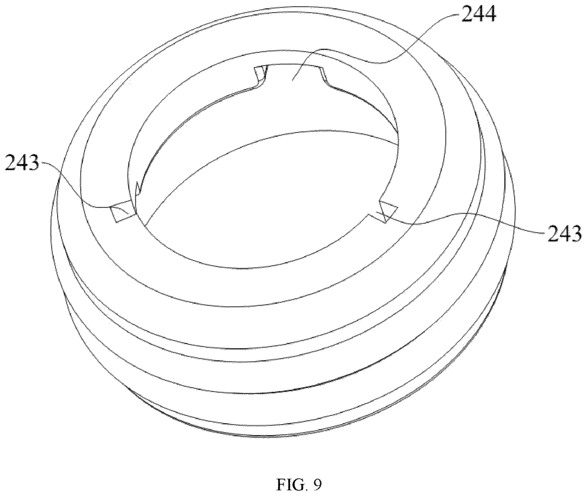

When the first sealing element 242 completely covers an outlet end of the groove 215, the first vent channel further includes a first notch 243 provided on the first sealing element 242, and the first notch 243 is in communication with the groove 215 and the liquid storage cavity 121. Referring to FIG. 2, FIG. 3, and FIG. 9, to prevent the first sealing element 242 from being assembled in a wrong position in an assembly process, causing the first notch 243 to be misaligned with the outlet end of the groove 215, a first limiting structure is further arranged on the first sealing element 242, and the first limiting structure may prevent the first sealing element 242 from being displaced relative to the porous liquid guide element 21. Correspondingly, a first fitting portion that matches the first limiting structure is further arranged on the porous liquid guide element 21. Through a limiting action between the first limiting structure and the first fitting portion, precise positioning between the first sealing element 242 and the porous liquid guide element 21 may be implemented. Specifically, the first limiting structure includes a fitting groove 244 provided on the first sealing element 242, and the first fitting portion includes one convex point 216 arranged on the porous liquid guide element 21. The first sealing element 242 may be successfully assembled only when the fitting groove 244 on the first sealing element the first sealing element 242 is aligned with the convex point 216 on the porous liquid guide element 21. To further expand a vent capacity of the groove 215, two grooves 215, namely, the first groove 2153 and the second groove 2154, are provided on the porous liquid guide element 21. Correspondingly, two first notches 243 and two grooves 215 are also provided on the first sealing element 242 for matching ventilation. Further, the first fitting portion is arranged between the first groove 2153 and the second groove 2154 on the porous liquid guide element 21, so that the two grooves 215 on the porous liquid guide element 21 and the two first notches 243 on the first sealing element 242 may be completely matched and be in communication with each other.

Further, a plurality of matching structures for the porous liquid guide element 21 are also arranged on the third sealing element 26, such as a position of the vent hole 262 on the third sealing element 26 and positions of the two lead posts 265. To enable the third sealing element 26 to be mounted according to a predetermined mounting position, a second limiting structure is further arranged on the third sealing element 26, and a second fitting portion is arranged on the bracket 23. Through interaction between the second limiting structure and the second fitting portion, precise positioning between the third sealing element 26 and the porous liquid guide element 21, and between the third sealing element 26 and the first sealing element 242 may be implemented. Specifically, the second limiting structure on the third sealing element 26 includes convex edges 266 arranged on two sides of the third sealing element 26. The second fitting portion on the bracket 23 includes two second notches 234 provided at an end portion of the bracket 23. The two convex edges 266 on the third sealing element 26 may only be assembled by aligning with the two second notches 234 on the bracket 23.

The groove 215 structure is provided on the porous liquid guide element 21, and therefore, in a demoulding process, the porous liquid guide element 21 may only be demoulded from two sides on which the two grooves 215 are located. To facilitate smooth formation of the porous liquid guide element 21, in a process in which the conductive pins 221 at the two ends of the heating element 22 and the porous liquid guide element 21 are combined, the conductive pins 221 need to be opened at a specific angle to be smoothly inserted into the inner wall of the porous liquid guide element 21. Therefore, the conductive pins 221 of the formed heating element 22 are not arranged parallel to an extending direction of an axis of the porous liquid guide element 21. A specific opening angle of the conductive pins 221 may be optimized and adjusted according to the injection molding process.

It should be noted that, the specification of this application and the accompanying drawings thereof illustrate preferred embodiments of this application, but this application is not limited to the embodiments described in the specification. Further, a person of ordinary skill in the art may make improvements or variations according to the foregoing descriptions, and such improvements and variations shall all fall within the protection scope of the appended claims of this application.

## Claims

1. An atomizer (100), comprising:
a housing, wherein a liquid storage cavity (121) is defined and formed in the housing, and the liquid storage cavity (121) is configured to store a liquid substrate;
a porous liquid guide element (21), configured to comprise a hollow tubular structure, wherein the porous liquid guide element (21) comprises a first section (211) and a second section (212) that are arranged in a longitudinal direction of the porous liquid guide element (21), an outer diameter of the first section (211) is less than an outer diameter of the second section (212), and a step surface (213) is formed as a transition between the second section (212) and the first section (211);
a heating element (22), fixed on the porous liquid guide element (21), and configured to atomize the liquid substrate;
a bracket (23), comprising an accommodating cavity (235), wherein the porous liquid guide element (21) is accommodated in the accommodating cavity (235), and a hole (231) is provided on the bracket (23), and is configured to guide the liquid substrate to enter the accommodating cavity (235), to allow the liquid substrate to be absorbed by the porous liquid guide element (21); and
a first sealing element (242), wherein at least a part of the first sealing element (242) surrounds an outer side surface of the second section (212), to provide a seal between an inner wall surface of the accommodating cavity (235) and the outer side surface of the second section (212).

2. The atomizer (100) according to claim 1, wherein the heating element (22) is arranged extending in the longitudinal direction of the porous liquid guide element (21), and the heating element (22) is limited to extend in a longitudinal extension length range of the first section (211) of the porous liquid guide element (21).

3. The atomizer (100) according to claim 1, further comprising a second sealing element (241), wherein the second sealing element (241) at least partially surrounds an outer side surface of an end portion of the first section (211) facing away from the second section (212).

4. The atomizer (100) according to claim 3, wherein a liquid guide cavity (232) is defined and formed between an inner wall surface of the bracket (23) and a part of an outer wall surface of the porous liquid guide element (21) that is not surrounded by the first sealing element (242) and the second sealing element (241), and the liquid substrate in the liquid storage cavity (121) is capable of entering the liquid guide cavity (232) through the hole.

5. The atomizer (100) according to claim 1, wherein a wall thickness of the first section (211) is less than a wall thickness of the second section (212).

6. The atomizer (100) according to claim 5, wherein the wall thickness of the first section (211) ranges from 0.5 mm to 1.2 mm, and the wall thickness of the second section (212) ranges from 1.2 mm to 3 mm.

7. The atomizer (100) according to claim 1, wherein the accommodating cavity (235) of the bracket (23) comprises a first part and a second part, an inner diameter of the first part is less than an inner diameter of the second part, the first part of the accommodating cavity (235) is configured to accommodate the first section (211) of the porous liquid guide element (21), and the second part of the accommodating cavity (235) is configured to accommodate the second section (212) of the porous liquid guide element (21).

8. The atomizer (100) according to claim 1, wherein a first vent channel is formed between the first sealing element (242) and the porous liquid guide element (21), and the first vent channel is configured to guide an external airflow to the liquid storage cavity (121).

9. The atomizer (100) according to claim 8, wherein the first vent channel comprises at least one groove (215) extending on the outer side surface of the second section (212) of the porous liquid guide element (21), and the groove (215) is in communication with the liquid storage cavity (121).

10. The atomizer (100) according to claim 9, wherein the groove (215) extends from a bottom end surface of the second section (212) to the step surface (213), wherein the step surface (213) is close to the liquid storage cavity (121).

11. The atomizer (100) according to claim 10, wherein the groove (215) extends non-linearly in the longitudinal direction.

12. The atomizer (100) according to claim 11, wherein the groove (215) extends in a substantially Z shape or S shape.

13. The atomizer (100) according to claim 10, 11, or 12, wherein the groove (215) comprises a first groove segment (2151) and a second groove segment (2152) in communication with each other, a width of the second groove segment (2152) is greater than a width of the first groove segment (2151), and the second groove segment (2152) is in communication with the liquid storage cavity (121).

14. The atomizer (100) according to claim 10, wherein the first vent channel further comprises a first notch (243) provided on the second sealing element (241), and the first notch (243) is in communication with the groove (215) and the liquid storage cavity (121).

15. The atomizer (100) according to claim 8, wherein when the porous liquid guide element (21) and the first sealing element (242) are accommodated in the accommodating cavity (235), the first vent channel corresponds to a position of the hole on the bracket (23) in the longitudinal direction.

16. The atomizer (100) according to claim 15, wherein a first limiting structure is arranged on the first sealing element (242), and the first limiting structure is configured to provide a stop to prevent the porous liquid guide element (21) from rotating relative to the first sealing element (242).

17. The atomizer (100) according to claim 16, wherein a first fitting portion that matches the first limiting structure is further arranged on the porous liquid guide element (21), and the first fitting portion protrudes from the step surface (213).

18. The atomizer (100) according to claim 1, further comprising a third sealing element (26) at least partially accommodated in the bracket (23), and the third sealing element (26) abuts against the porous liquid guide element (21) in the longitudinal direction.

19. The atomizer (100) according to claim 18, wherein the third sealing element (26) further comprises a second limiting portion, configured to cooperate with the bracket (23) to prevent the third sealing element (26) from rotating relative to the bracket (23).

20. The atomizer (100) according to claim 19, wherein a second fitting portion that matches the second limiting portion is arranged on the bracket (23), and the second fitting portion comprises a second notch (234).

21. The atomizer (100) according to claim 18, wherein the third sealing element (26) comprises a cavity (261) adjacent to an end surface of the second section (212), and the cavity (261) is configured to receive condensate.

22. The atomizer (100) according to claim 18, wherein the atomizer (100) further comprises an electrical connecting member (133) electrically connected to the heating element (22), a baffle plate (264) is arranged on the third sealing element (26), and the baffle plate (264) extends from an end portion of the third sealing element (26) toward the electrical connecting member (133).

23. The atomizer (100) according to claim 18, wherein a vent hole (262) is provided on the third sealing element (26).

24. The atomizer (100) according to claim 23, wherein at least a part of a side surface of the third sealing element (26) is concave, to enable an air guide cavity (263) to be formed between the third sealing element (26) and an inner wall of the bracket (23), and the air guide cavity (263) is in communication with the vent hole (262).

25. The atomizer (100) according to claim 1, wherein two ends of the heating element (22) are connected to conductive pins (221), and the conductive pins (221) extend out from inside of the porous liquid guide element (21), wherein an extension direction of each of the conductive pins (221) is arranged non-parallel to an axis of the porous liquid guide element (21).

26. The atomizer (100) according to claim 1, wherein the first sealing element (242) comprises a sleeve portion and a radial extension portion, and the radial extension portion covers the step surface (213).

27. A ceramic heating body (27) used in an aerosol generating apparatus, comprising a porous liquid guide element (21) configured to store and transfer a liquid substrate and a heating element (22) combined on the porous liquid guide element (21), wherein the porous liquid guide element (21) is configured to comprise a hollow tubular structure, the porous liquid guide element (21) comprises a first section (211) and a second section (212) that are arranged in a longitudinal direction, an outer diameter of the first section (211) is less than an outer diameter of the second section (212), and a step surface (213) is formed as a transition between the second section (212) and the first section (211); and the heating element (22) is configured to atomize the liquid substrate.

28. The ceramic heating body (27) according to claim 27, wherein at least one groove (215) is provided on an outer wall of the porous liquid guide element (21).

29. An aerosol generating apparatus, comprising the atomizer (100) according to claims 1 to 26, and a power supply assembly (200) that provides electric drive for the atomizer (100).
